# EUROPÄISCHE PATENTSCHRIFT

(11) **EP 0 387 363 B1**
(45) Veröffentlichungstag und Bekanntmachung des Hinweises auf die Patenterteilung: **01.06.1994**
(21) Anmeldenummer: 89104401.8
(22) Anmeldetag: 13.03.1989
(51) Int. Cl.: A61N 1/365, A61N 1/372

(54) **Implantierbares medizinisches Gerät zur Stimulation eines physiologischen Vorganges eines Lebewesens mit an die körperliche Aktivität des Lebewesens anpassbarer Stimulationsintensität**
Implantable stimulator whose intensity of stimulation of a physiologic event of living beings is adapted to their physical actvity
Stimulateur implantable dont l'intensité de stimulation d'un événement physiologique d'un être vivant est adaptée à son activité physique

(43) Veröffentlichungstag der Anmeldung: 19.09.1990
(73) Patentinhaber: Pacesetter AB, 171 95 Solna (SE)
(72) Erfinder: Nigram, Indra, Dipl.-Ing., S-172 45 Sundbyberg (SE)
(74) Vertreter: Lettström, Richard Wilhelm

(56) Entgegenhaltungen:
- EP-A- 0 194 224
- EP-A- 0 225 839
- EP-A- 0 228 985
- GB-A- 2 026 870

## Beschreibung

Die Erfindung betrifft ein in den Körper eines Lebewesens implantierbares medizinisches Gerät mit Mitteln zum Stimulieren eines physiologischen Vorgangs des Lebewesens mittels einem Stimulationssignals einstellbarer Stimulationsgröße und selbsttätigen Stellmitteln zur Anpassung der Stimulationsgröße an die körperliche Aktivität des Lebewesens, die die Stimulationsgröße anhand eines der körperlichen Aktivität des Lebewesens entsprechenden Signales einer Sensoreinrichtung einstellen, wobei dem Signal der Sensoreinrichtung entsprechende Daten von dem medizinischen Gerät zu einer getrennten Datenverarbeitungseinrichtung telemetrisch übertragbar sind und das Gerät einen wahlweise im Schreib- oder Lesemodus betreibbaren Speicher enthält, dem im Schreibmodus diese Daten zur Speicherung zuführbar sind, welche im Lesemodus zur telemetrischen Übertragung zu der Datenverarbeitungseinrichtung abrufbar sind. Der Begriff Stimulationsgröße soll hier umfassend verstanden werden, d.h., sowohl die Dauer als auch die Häufigkeit, Folgefrequenz, Amplitude etc., mit der die Mittel zur Stimulation tätig werden, sollen einzeln und/oder in Kombination als Mass für die Stimulationsintensität verstanden werden.

So ein Gerät ist aus EP-A-0 225 839 vorbekannt.

Es ist auch ein derartiges Gerät bekannt, das als Herzschrittmacher ausgebildet ist, bei dem die Stimulationsfrequenz, mit der der Herzschrittmacher das Herz beim Ausbleiben von natürlichen Herzschlägen stimuliert, in Abhängigkeit von der körperlichen Aktivität des Lebewesens einstellbar ist. Dabei ist als Sensoreinrichtung ein Temperatursensor vorhanden, der die Körpertemperatur des den Herzschrittmacher tragenden Lebewesens als Mass für die jeweils vorhandene körperliche Aktivität erfasst. Da die Stellmittel, die die selbsttätige Anpassung der Stimulationsfrequenz an die körperliche Aktivität des Lebewesens vornehmen, programmierbar sind, besteht die Möglichkeit, die individuellen Bedürfnisse des Lebewesens durch eine entsprechende Programmierung der Stellmittel zu berücksichtigen. Dies geschieht im Falle des bekannten Herzschrittmachers, indem sich das den Herzschrittmacher tragende Lebewesen einer definierten körperlichen Belastung unterzieht. Dem zeitlichen Verlauf der dabei gemessenen Körpertemperatur entsprechende Daten werden telemetrisch zu einem Kleincomputer übertragen, der diese speichert. Es besteht nun die Möglichkeit, die Reaktion der Stellmittel auf das gespeicherte, einer definierten körperlichen Belastung entsprechende Temperaturprofil mittels des entsprechend programmierten Kleincomputers zu simulieren. Dabei können verschiedene Programmierungen der Stellmittel simuliert werden, um eine den individuellen Bedürfnissen des jeweiligen Lebewesens angepasste Programmierung auffinden zu können. Ist dies geschehen, werden die der entsprechenden Programmierung der Stellmittel entsprechenden Daten telemetrisch von der Datenverarbeitungseinrichtung zu dem Herzschrittmacher übertragen.

In der Praxis wird es als nachteilig empfunden, dass sich das Lebewesen jedesmal, wenn die Programmierung der Stellmittel überprüft oder verändert werden soll, erneut einer körperlichen Belastung unterziehen muss, um die zur Simulation der Funktion der Stellmittel erforderlichen Daten zu gewinnen. Ausserdem wird es als nachteilig empfunden, dass sich das Lebewesen, während es sich der körperlichen Belastung unterzieht, in unmittelbarer Nähe des Kleincomputers aufhalten oder eine über Kabel mit dem Kleincomputer verbundene Empfangseinrichtung tragen muss, so dass die Bewegungsfreiheit des Lebewesens eingeschränkt ist, während es sich der körperlichen Beanspruchung unterzieht.

Der Erfindung liegt die Aufgabe zugrunde, ein Gerät der eingangs genannten Art so auszubilden, dass während desjenigen Zeitraumes, bezüglich dessen dem zeitlichen Verlauf des Signals der Sensoreinrichtung entsprechende Daten gewonnen werden, keine nennenswerte Einschränkungen der Bewegungsfreiheit des Lebewesens auftreten.

Nach der Erfindung wird diese Aufgabe dadurch gelöst, dass die Daten den zeitlichen Verlauf des Signals der Sensoreinrichtung während eines definierten Zeitraumes darstellen. Wenn sich also das Lebewesen, in dessen Körper ein erfidnungsgemässes Gerät implantiert ist, während des definierten Zeitraumes einer bestimmten körperlichen Beanspruchung unterzieht, werden die dem zeitlichen Verlauf des Signales der Sensoreinrichtung während dieses Zeitraumes entsprechenden Daten in dem Speicher gespeichert, wenn dieser zuvor in den Schreibmodus geschaltet wurde. Sie können von dort zu einem beliebiegen späteren Zeitpunkt nach Schalten in den Lesemodus telemetrisch zu der Datenverarbeitungseinrichtung übertragen werden. Es wird somit deutlich, dass im Falle des erfindungsgemässen Gerätes keinerlei Einschränkung der Bewegungsfreiheit des Lebewesens erforderlich ist, während sich dieses der körperlichen Beanspruchung unterzieht. Ausserdem besteht die Möglichkeit, die dem zeitlichen Verlauf des Signals der Sensoreinrichtung entsprechenden Daten beliebig oft aus dem Speicher des Gerätes abzurufen, so dass sich das Lebewesen nicht jedesmal, wenn derartige Daten benötigt werden, einer erneuten körperlichen Beanspruchung unterziehen muss. Dies wird vielmehr nur dann erforderlich sein, wenn die gespeicherten Daten aus einer soweit zurückliegenden Zeit stammen, dass damit zu rechnen ist, dass sie der aktuellen körperlichen Verfassung des Lebewesens nicht mehr entsprechen.

Gemäss einer bevorzugten Variante der Erfindung ist vorgesehen, dass der Speicher, der übrigens mittels eines dem Gerät telemetrisch zuführbaren Signal wahlweise in den Schreib- oder Lesemodus schaltbar ist, eine Vielzahl einzeln adressierbarer Speicherzellen besitzt und dass Adressierungsmittel für die Speicherzellen vorgesehen sind, die nach dem Schalten des Speichers in den Schreibmodus die Speicherzellen in einer definierten Folge jeweils einmal adressieren und nach dem Schalten des Speichers in den Lesemodus die Speicherzellen erneut in der definierten Folge adressieren. In diesem Falle ist also der Beginn des definierten Zeitraumes, bezüglich dessen dem Signal der Sensoreinrichtung entsprechende Daten gespeichert werden, der Zeitpunkt, zu dem der Speicher mittels eines dem Gerät telemetrisch zugeführten Signales in den Schreibmodus geschaltet wird, so dass der Beginn des Zeitraumes praktisch frei wählbar ist. Ebenso ist der Zeitpunkt, zu dem die gespeicherten Daten zur telemetrischen Übertragung aus dem Speicher abgerufen werden nach Belieben wählbar, indem zu dem gewünschten Zeitpunkt der Speicher mittels eines dem Gerät telemetrisch zugeführten Signales in den Lesemodus geschaltet wird.

Es kann jedoch auch zweckmässig sein, wenn die Adressierungsmittel nach dem Schalten des Speichers in den Schreibmodus die Speicherzellen in einem Adressierungszyklus fortlaufend in einer definierten Folge adressieren und nach dem Schalten des Speichers in den Lesemodus beginnend mit derjenigen Speicherzelle, die der im Schreibmodus zuletzt adressierten Speicherzelle folgt, sämtliche Speicherzellen entsprechend der definierten Folge jeweils einmal adressiert. In diesem Falle sind in dem Speicher also jeweils Daten gespeichert, die dem zeitlichen Verlauf des Signals der Sensoreinrichtung während des sich ausgehend vom Zeitpunkt des Schaltens des Speichers in den Lesebetrieb zurückerstreckenden definierten Zeitraumes entsprechen.

Gemäss einer weiteren bevorzugten Ausführungsform der Erfindung ist das Gerät als Herzschrittmacher ausgebildet, wobei die Mittel zum Stimulieren eines physiologischen Vorganges des Lebewesens dessen Herztätigkeit stimulieren und die Stellmittel die Stimulationsfrequenz, mit der das Herz im Bedarfsfalle stimuliert wird, an die körperliche Aktivität des Lebewesens anpassen.

Besonders vorteilhaft ist es, wenn gemäss einer Variante der Erfindung die Stellmittel mittels der Datenverarbeitungseinrichtung telemetrisch programmierbar sind. Dieser Vorteil kommt insbesondere in einer erfindungsgemässen medizinischen Einrichtung zum Tragen, die ein erfindungsgemässes medizinisches Gerät und eine Datenverarbeitungseinrichtung umfasst und dadurch gekennzeichnet ist, dass die Funktion der Stellmittel unter Zugrundelegung von dem zeitlichen Verlauf des Signales der Sensoreinrichtung entsprechenden in dem Speicher des Gerätes gespeicherten Daten mittels der Datenverarbeitungseinrichtung zur Ermittelung der Stimulationsintensität in Abhängigkeit von der dem zeitlichen Verlauf des Signales der Sensoreinrichtung entsprechenden körperlichen Aktivität des Lebewesens simulierbar ist und dass die Datenverarbeitungseinrichtung Mittel zur insbesondere graphischen Darstellung der Stimulationsintensität als Funktion der körperlichen Aktivität aufweist. In diesem Falle ist es nämlich möglich, verschiedene Programmierungen der Stellmittel zunächst zu simulieren, um dann diejenige Programmierung, die den Bedürfnissen des das Gerät tragenden Lebewesens am besten entspricht, telemetrisch vorzunehmen.

Weitere Vorteile und Merkmale der Erfindung ergeben sich aus der Erläuterung eines Ausführungsbeispiels anhand der beigefügten Figur, die das Blockschaltbild eines erfindungsgemäss ausgebildeten Herzschrittmachers zeigt.

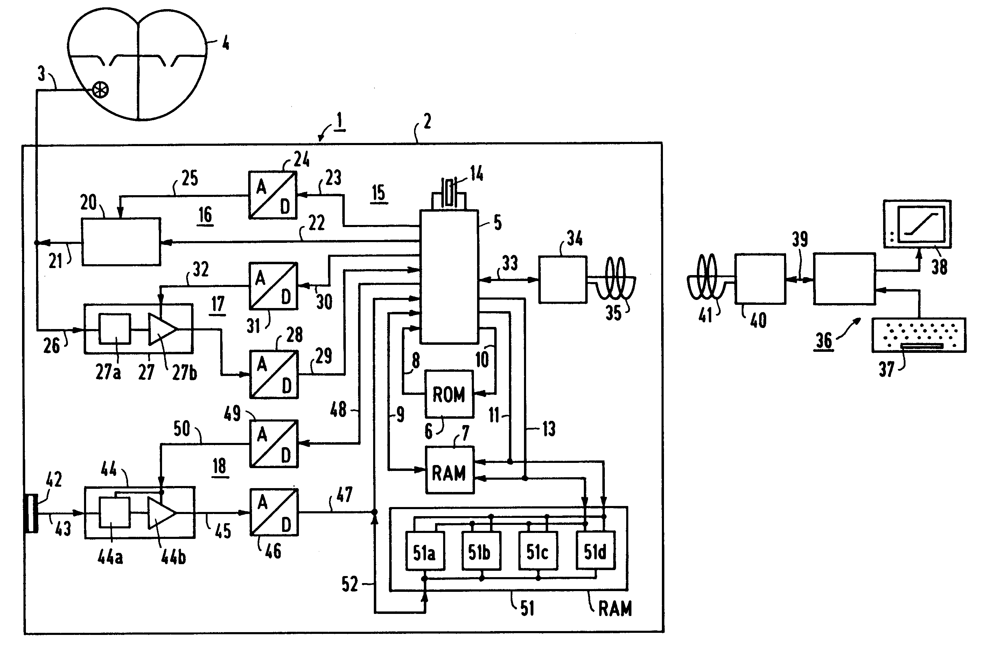

In FIG 1 ist die Erfindung anhand eines insgesamt mit (1) bezeichneten Herzschrittmachers dargestellt. Die Bauteile des Herzschrittmachers (1) sind in einem schematisch angedeuteten hermetisch dichten Gehäuse (2) aufgenommen. Von dem im VVI-Mode arbeitenden Herzschrittmacher (1) führt eine Elektrode (3) zu einem Herz (4) eines Lebewesens und ist dort in das Ventrikel eingepflanzt.

Der Herzschrittmacher (1) umfasst unter anderem einen Mikroprozessor (5), dem ein Nur-Lese-Speicher (ROM) (6) und ein Schreib-Lese-Speicher (RAM) (7) zugeordnet sind, die über Datenleitungen (8,9) und Adressleitungen (10,11) mit dem Mikroprozessor (5) in Verbindung stehen. Zu dem RAM (7) führt von dem Mikroprozessor (5) ausserdem eine Leitung (13), die zum Umschalten des RAM (7) von Schreib-auf Lesebetrieb und umgekehrt dient. In dem ROM (6) ist ein Programm gespeichert, mittels dessen sämtliche Funktionen des Herzschrittmachers (1) gesteuert werden. Wenn also im folgenden die Rede davon ist, dass der Mikroprozessor (5) eine bestimmte Funktion ausführt, so ist darunter zu verstehen, dass der Mikroprozessor (5) unter Ausführung des im ROM (6) gespeicherten Programmes bei Heranziehung von im RAM (7) befindlichen Daten und ihm anderweitig, z.B. über eine Eingangs-Beschaltung, zugeführter Daten zur Ausführung der jeweiligen Funktion tätig wird.

Mit dem Mikroprozessor (5) ist ein Quarz (14) verbunden, der zur Erzeugung der für den Betrieb des Mikroprozessors (5) erforderlichen Taktsignale dient und ausserdem die Zeitreferenz für den Betrieb des Herzschrittmachers (1) darstellt.

Der Mikroprozessor (5) des Herzschrittmachers (1) besitzt eine insgesamt mit (15) bezeichnete Eingangs/Ausgangs-Beschaltung, die mehrere Kanäle (16,17,18) aufweist.

Der Kanal (16) dient dazu, das Herz (4) erforderlichenfalls mit Stimulationsimpulsen zu versorgen. Der Kanal (16) besitzt daher einen Stimulationsimpuls-Generator (20), dessen Ausgangsleitung (21) mit der Elektrode (3) verbunden ist. Der Stimulationsimpuls-Generator (20) ist über eine Leitung (22), die mit einem entsprechenden Ausgang des Mikroprozessors (5) verbunden ist, zur Abgabe eines Stimulationsimpulses aktivierbar. Digitale Daten, die die Form der Stimulationsimpulse, z.B. deren Amplitude und Dauer, betreffen, gelangen von dem Mikroprozessor (5) über eine Leitung (23) zu einer Digital/Analog-Schnittstelle (24), die den Stimulationsimpuls-Generator (20) über eine Steuerleitung (25) den digitalen Daten entsprechende analoge Steuersignale zuführt, die den Stimulationsimpuls-Generator (20) derart einstellen, dass er bei Bedarf Stimulationsimpulse der gewünschten Form erzeugt.

Der Kanal (17) besitzt eine über eine Eingangsleitung (26) ebenfalls mit der Elektrode (3) verbundene Signalaufbereitungsschaltung (27), die dazu dient, ein mittels der Elektrode (3) vom Herzen (4) abgenommenes der Aktivität des Herzens entsprechendes elektrisches Signal zu filtern und zu verstärken. Die Signalaufbereitungsschaltung (27) umfasst daher ein Filter (27a) und einen Verstärker (27b). Vom Ausgang der Signalaufbereitungsschaltung (27) gelangt das aufbereitete Signal zu einem Analog/Digital-Wandler (28). Von diesem gelangen über eine Leitung (29) digitale Daten zu einem entsprechenden Eingang des Mikroprozessors (5), die dem Verlauf des am Ausgang der Signalaufbereitungsschaltung (27) elektrischen Signales entsprechen, das seinerseits die elektrische Aktivität des Herzens (4) wiederspiegelt. Der Mikroprozessor (5) ist über eine Leitung (30) mit einer Digital/Analog-Schnittstelle (31) verbunden, die ihr von dem Mikroprozessor (5) zugeführte digitale Daten als entsprechende analoge Signale über eine Steuerleitung (32) an die Signalaufbereitungsschaltung (27) weitergibt. Die digitalen Daten bzw. die entsprechenden analogen Signale dienen dazu, z.B. den Verstärkungsfaktor des Verstärkers (27b) einzustellen bzw. den Verstärker (27b) vollständig zu sperren.

Die über die Leitung (29) dem Mikroprozessor (5) zugeführten digitalen Daten analysiert dieser daraufhin, ob in dem der Aktivität des Herzens (4) entsprechenden elektrischen Signal Ereignisse enthalten sind, die dem Auftreten eines natürlichen Herzschlages entsprechen. Detektiert der Mikroprozessor (5) einen natürlichen Herzschlag oder aktiviert er über die Leitung (22) den Stimulationsimpuls-Generator (20) zur Abgabe eines Stimulationsimpulses, beginnt der Mikroprozessor (5) als Zähler zu arbeiten und beginnt eine Anzahl von aus der Schwingung des Quarzes (14) abgeleiteten Taktimpulse abzuzählen, die einem zwischen einer oberen und einer unteren Grenze einstellbaren Zeitintervall entspricht. Das jeweils eingestellte Zeitintervall bestimmt diejenige Stimulationsfrequenz mit der das Herz (4) beim Ausbleiben natürlicher Herzschläge stimuliert wird. Gelangen während dieses Zeitintervalles über den Kanal (17) keine Daten zu dem Mikroprozessor (5), die dieser als natürlichen Herzschlag detektiert, aktiviert der Mikroprozessor (5) bei Ablauf des Zeitintervalls über die Leitung (22) den Stimulationsimpuls-Generator (20). Im Anschluss an die Abgabe eines Stimulationsimpulses beginnt der Mikroprozessor (5) erneut eine Anzahl von Taktimpulsen abzuzählen, die dem jeweils eingestellten, die Stimulationsfrequenz bestimmenden Zeitintervall entspricht. Detektiert der Mikroprozessor (5) dagegen während des Laufs des jeweils eingestellten, die Stimulationsfrequenz bestimmenden Zeitintervalls einen natürlichen Herzschlag, bricht er den beschriebenen Zählvorgang ab, sofern ein weiteres Zeitintervall, die sogenannte Refraktärzeit, abgelaufen ist, und beginnt den beschriebenen Zählvorgang von neuem.

Die Refraktärzeit, die grundsätzlich kürzer ist als das zwischen beispielsweise 400 und 2000 ms einstellbare, die Stimulationsfrequenz bestimmende Zeitintervall, dauert zwischen etwa 250 und 450 ms (einstellbar). Die Refraktärzeit unterteilt sich in eine absolute Refraktärzeit mit einer festen Dauer von gewöhnlich 125 ms und eine relative Refraktärzeit, auf die der restliche Teil der gesamten jeweils eingestellten Refraktärzeit entfällt. Die Refraktärzeit beginnt jeweils gleichzeitig mit dem die Stimulationsfrequenz bestimmenden Zeitintervall zu laufen und wird von dem Mikroprozessor (5) durch den gleichen Zählvorgang ermittelt, der auch zur Ermittlung des die Stimulationsfrequenz bestimmenden Zeitintervalles dient. Während der absoluten Refraktärzeit ist im Kanal (17) der Verstärker (27b) der Signalaufbereitungsschaltung (27) vollständig gesperrt, was dadurch erreicht wird, dass der Mikroprozessor (5) den Verstärker (27b) über die Leitung (30), die Digital/Analog-Schnittstelle (31) und die Steuerleitung (32) mit einem entsprechenden Steuersignal beaufschlagt. Infolge der vollständigen Sperrung des Verstärkers (27b) ist mittels des Mikroprozessors (5) während der Dauer der absoluten Refraktärzeit keinerlei Detektion möglich. Nach Ablauf der absoluten Refraktärzeit aktiviert der Mikroprozessor (5) den Verstärker (27b) wieder, so dass er in der Lage ist, natürliche Herzschläge zu detektieren. Detektiert der Mikroprozessor (5) während der relativen Refraktärzeit einen natürlichen Herzschlag, bricht er im Gegensatz zu einer Detektion nach Ablauf der Refraktärzeit den Zählvorgang zur Ermittlung des jeweils eingestellten, die Stimulationsfrequenz bestimmenden Zeitintervalles nicht ab, sondern führt ihn fort und schliesst ihn mit einer Aktivierung des Stimulationsimpuls-Generators (20) ab. Allerdings setzt der Mikroprozessor (5) nach der Detektion eines natürlichen Herzschlages nochmals die volle Refraktärzeit in Gang. Hierdurch wird erreicht, dass im Falle von Hochfrequenzstörungen, die zu Fehldetektionen führen, unabhängig vom Auftreten natürlicher Herzschläge Stimulationsimpulse mit der durch das jeweils eingestellte Zeitintervall bestimmten Stimulationsfrequenz erzeugt werden. Auch wenn die spontane Herzschlagfrequenz so hoch liegt, dass das Auftreten natürlicher Herzschläge jedesmal innerhalb der relativen Refraktärzeit erfolgt, erfolgt eine Abgabe von Stimulationsimpulsen mit der durch das jeweils eingestellte Zeitintervall bestimmten Stimulationsfrequenz, und zwar bis die spontane Herzschlagfrequenz unter eine Frequenz zurückgefallen ist, deren Periodendauer der jeweils eingestellten Refraktärzeit entspricht. Mittels dieser Funktion ist die Beendigung bestimmter Wiedereintrittstachykardien möglich.

Der Mikroprozessor (5) ist über eine Leitung (33) mit einem Telemetrieschaltkreis (34) verbunden, an den eine Sende/Empfangs-Spule (35) angeschlossen ist. Der Herzschrittmacher (1) ist somit in der Lage, mit einer externen Datenverarbeitungseinrichtung, nämlich einem Personal Computer (PC) (36) mit einer Tastatur (37) und einem Monitor (38), Daten auszutauschen, da der PC (36) über eine Leitung (39) mit einem zweiten Telemetrieschaltkreis (40) verbunden ist, an den wieder eine Sende/Empfangs-Spule (41) angeschlossen ist. Zum Datenaustausch zwischen dem Herzschrittmacher (1) und dem PC (36) wird die Sende/Empfangs-Spule (41) des zu dem PC (36) gehörigen Telemetrieschaltkreises (40) so auf der Körperoberfläche des den Herzschrittmacher (1) tragenden Lebewesens positioniert, dass sie mit der Sende-Empfangs-Spule (35) des Herzschrittmachers (1) induktiv gekoppelt ist. Es besteht dann die Möglichkeit, dem PC (36) die in dem ROM (6) und dem RAM (7) befindlichen Daten zur Überprüfung bzw. Überprüfung und Veränderung zuzuführen. Ausserdem besteht die Möglichkeit, dem RAM (7) des Herzschrittmachers (1) von dem PC (36) her veränderte bzw. zusätzliche Daten zuzuführen.

Der Kanal (18) der Eingangs/Ausgangs-Schaltung (15) des Mikroprozessors (5) dient dazu, dem Mikroprozessor (5) Daten zur Verfügung zu stellen, die es diesem anhand des in dem ROM (6) gespeicherten Programmes gestatten, das die Stimulationsfrequenz bestimmende Zeitintervall an die köperliche Aktivität des den Herzschrittmacher (1) tragenden Lebewesens anzupassen. Zu diesem Zweck ist ein piezoelektrischer Druck-Sensor (42) vorgesehen, der mit der Wandung des Gehäuses (2) verbunden ist. Während körperlicher Aktivitäten des Lebewesens entstehen durch die Bewegung der Muskeln und dergleichen mechanische Schwingungen im Körper des Lebewesens, die sich als Druckwellen in dem Körper des Lebewesens ausbreiten und von dem piezoelektrischen Sensor (42) aufgenommen und in elektrische Signale umgewandelt werden. Diese Signale, deren Amplitude mit zunehmender körperlicher Aktivität ebenfalls zunimmt, gelangen über eine Leitung (43) zu einer Signalaufbereitungsschaltung (44), die ein Filter (44a) mit nachfolgendem Verstärker (44b) enthält. Das Ausgangssignal der Signalaufbereitungsschaltung (44) gelangt über eine Leitung (45) zu einem Analog/Digital-Wandler (46), z.B. einem 8-bit-Wandler, dessen digitale Ausgangssignale über eine Leitung (47) zu dem Mikroprozessor (5) gelangen. Der Mikroprozessor (5) ist über eine Leitung (48) mit einer Digital/Analog-Schnittstelle (49) verbunden, die ihr von dem Mikroprozessor (5) zugeführte digitale Daten als entsprechende analoge Signale über eine Steuerleitung (50) an die Signalaufbereitungsschaltung (44) weitergibt. Die digitalen Daten bzw. die diesen entsprechenden analogen Signale dienen dazu, z.B. den Verstärkungsfaktor des Verstärkers (44b) einzustellen oder die Charakteristik des Filters (44a) zu verändern. In Abhängigkeit von dem zeitlichen Verlauf des von dem piezoelektrischen Sensor (42) stammenden Signals bzw. der entsprechenden digitalen Daten verändert der Mikroprozessor (5) in ähnlicher Weise wie dies in der EP-A-0 080 348 beschrieben ist, das die Stimulationsfrequenz bestimmende Zeitintervall derart, dass dieses mit zunehmender körperlicher Aktivität verkürzt wird. Dies vollzieht sich zwischen einer unteren Grenze (Ruhepuls) und einer oberen Grenze (maximale Herzschlagfrequenz), die den Bedürfnissen des jeweiligen Lebewesens entsprechend gewählt werden.

Der Herzschrittmacher (1) besitzt ausserdem einen zusätzlichen Schreib-Lese-Speicher (RAM) (51), der ebenso wie das RAM (7) über die Adressleitung (11) und die Steuerleitung (13) mit dem Mikroprozessor (5) verbunden ist. Das RAM (51) ist in vier Speichersegmente (51a, 51b, 51c, 51d) unterteilt, die mittels des Mikroprozessors (5) separat adressierbar sind.

Das RAM (51) ist vorgesehen, um die digitalen Ausgangsdaten des Analog/Digital-Wandlers (46) speichern zu können, die dem zeitlichen Verlauf des Signales des piezoelektrischen Sensors (42) während eines definierten Zeitraums entsprechen. Zu diesem Zweck schaltet der Mikroprozessor (5) das RAM (51), dessen Datenleitung (52) mit der Leitung (47) verbunden ist, über die Steuerleitung (13) zunächst in den Schreibmodus. Damit dies geschieht, muss durch geeignetes Betätigen der Tastatur (37) des PC (36) ein entsprechender Befehl telemetrisch zu dem Herzschrittmacher (1) übertragen werden. Die in dem RAM (51) befindlichen Daten können nach Schalten des RAM (51) in den Lesemodus jederzeit - es muss wieder eine geeignete Betätigung der Tastatur (37) des PC (36) vorausgehen - telemetrisch in das RAM (7) des PC (36) übertragen werden. Wenn wie im Falle des dargestellten Ausführungsbeispiels im Lesemodus die in dem RAM (51) gespeicherten Daten über die gleiche Leitung (47) zu dem Mikroprozessor (5) gelangen, über die diesem normalerweise die digitalen Ausgangsdaten des Analog/Digital-Wandlers (46) zugeführt sind, muss zur Vermeidung von Störungen sichergestellt sein, dass keine Daten vom Ausgang des Analog/Digital-Wandlers (46) auf die Leitung (47) gelangen, solange sich das RAM (51) im Lesemodus befindet. Dies kann z.B. geschehen, indem der Mikroprozessor (5) die Signalaufbereitungsschaltung (44) sperrt, indem er der Digital/Analog-Schnittstelle (49) entsprechende Daten zuführt. Es ist in nicht dargestellter Weise jedoch auch möglich, den Ausgang des Analog/Digital-Wandlers (46) von der Leitung (47) zu trennen oder den Analog/Digital-Wandler (46) zu sperren.

Wenn die in dem RAM (51) gespeicherten Daten aus einem Zeitraum stammen, in dem sich das Lebewesen einer definierten körperlichen Beanspruchung unterzogen hat, und der PC (36) derart programmiert ist, dass er in der Lage ist, die Einstellung des die Stimulationsfrequenz bestimmenden Zeitintervalles in der Weise zu simulieren, wie sie der Mikroprozessor (5) des Herzschrittmachers (1) bei einer gegebenen Programmierung vornehmen würde, besteht also die Möglichkeit, die Stimulationsfrequenz als Funktion der körperlichen Aktivität des Lebewesens auf dem Monitor (38) des PC (36) darzustellen.

Ein behandelnder Arzt ist dann in der Lage, zu überprüfen, ob die Programmierung des Herzschrittmachers (1) den Bedürfnissen des jeweiligen Lebewesens entspricht. Falls er Änderungen der Programmierung für erforderlich hält, kann er deren Auswirkungen zunächst auf dem PC (36) simulieren, bevor telemetrisch eine entsprechende Umprogrammierung des Herzschrittmachers (1) erfolgt.

Der erfindungsgemässe Herzschrittmacher (1) bietet den Vorteil, dass sich die Sende/Empfangs-Spule (41) des mit dem PC (36) verbundenen Telemetrieschaltkreises (40) nur dann in der Nähe des den Herzschrittmacher (1) tragenden Lebewesens befinden muss, wenn das RAM (51) in den Schreibmodus geschaltet bzw. wenn das RAM (51) in den Lesemodus geschaltet werden und die telemetrische Übertragung der gespeicherten Daten erfolgen soll. Während sich das den Herzschrittmacher (1) tragende Lebewesen einer körperlichen Beanspruchung unterzieht, bestehen also keinerlei Einschränkungen seiner Bewegungsfreiheit. Ausserdem stehen die in dem RAM (51) gespeicherten Daten jederzeit zur Verfügung, ohne dass sich das Lebewesen erneut einer körperlichen Beanspruchung unterziehen muss.

Wie bereits erwähnt wurde, ist das RAM (51) in vier Speichersegmente (51a,51b,51c,51d) unterteilt. Diese können in der zuvor für das gesamte RAM (51) beschriebenen Weise getrennt voneinander und zu unterschiedlichen Zeitpunkten zur Speicherung von Daten in den Schreibmodus geschaltet werden. Es besteht also die Möglichkeit, dem zeitlichen Verlauf des Signals des piezoelektrischen Sensors (42) entsprechende Daten für mehrere definierte Zeiträume zu speichern. Dies ist besonders dann vorteilhaft, wenn während der entsprechenden Zeiträume körperliche Beanspruchungen vorlagen, die hinsichtlich ihrer Intensität und ihres zeitlichen Verlaufes voneinander abweichen.

Das RAM (51) bzw. dessen Speichersegmente (51a,51b,51c, 51d) enthalten eine Vielzahl von mittels des Mikroprozessors (5) einzeln adressierbaren, eine bestimmte bit-Breite aufweisenden Speicherzellen. Die Speicherzellen des gesamten RAM (51) bzw. eines der Speichersegmente (51a,51b,51c,51d) werden nach dem Schalten in den Schreibmodus jeweils einmal in einer definierten Folge adressiert, wobei es sich bei der definierten Folge in der Regel um die Reihenfolge gemäss den Adressnummern handelt. Die Länge des definierten Zeitraumes, während dessen dem zeitlichen Verlauf des Signals des piezoelektrischen Sensors (42) entsprechende Daten speicherbar sind, entspricht also derjenigen Zeitdauer, die verstreicht, bis sämtliche Speicherzellen des gesamten RAM (51) bzw. eines Speichersegmentes (51a,51b,51c,51d) einmal adressiert sind. Nach dem Schalten in den Lesemodus werden die Speicherzellen des gesamten RAM (51) bzw. eines Speichersegmentes (51a,51b,51c,51d) wieder erneut in der definierten Folge adressiert, wobei gleichzeitig die telemetrische Übertragung der Daten zu dem PC (36) erfolgt.

Falls dies wünschenswert ist besteht jedoch auch die Möglichkeit, durch eine geeignete Betätigung der Tastatur (37) des PC (36) den Mikroprozessor (5) telemetrisch zu veranlassen, die eben beschriebene Adressierungsweise dahingehend zu ändern, dass sich die definierte Folge, in der er die Speicherzellen RAM (51) bzw. eines der Speichersegmente (51a,51b,51c,51d) nach dem Schalten in den Schreibmodus adressiert, zyklisch fortlaufend solange wiederholt, bis vom Schreib- in den Lesemodus übergegangen wird. Nach dem Schalten in den Lesemodus adressiert der Mikroprozessor (5) beginnend mit derjenigen Speicherzelle, die der im Schreibemodus zuletzt adressierten Speicherzelle folgt, sämtliche Speicherzellen entsprechend der definierten Folge jeweils einmal zur telemetrischen Übertragung der entsprechenden Daten zu dem PC (36). Im Falle der zuletzt beschriebenen Adressierungsweise sind also stets diejenigen Daten gespeichert, die einen definierten Zeitraum betreffen, der sich ausgehend von einem gegebenen Zeitpunkt zeitlich zurückerstreckt und dessen Zeitdauer wieder der zu der Adressierung der beteiligten Speicherzellen erforderlichen Zeit entspricht.

So kann beispielsweise das RAM (51) insgesamt 1024 Speicherzellen mit einer bit-Breite von jeweils 8 bit aufweisen, von denen jeweils 256 Speicherzellen auf jedes der Speichersegmente (51a,51b,51c,51d) entfallen. Wird das Signal des piezoelektrischen Sensors z.B. in Abständen von einer Sekunde abgetastet und mittels des Analog/Digital-Wandlers (46) in entsprechende digitale Daten umgesetzt, sind Daten bezüglich eines Zeitraumes von längstens ca. 17 Minuten speicherbar. Es besteht jedoch auch die Möglichkeit Daten aus vier verschiedenen Zeiträumen von jeweils ca. 4 Minuten Dauer in den einzelnen Speichersegmenten (51a,51b,51c,51d) zu speichern.

Erfolgt die Adressierung der Speicherzellen in der zuerst beschriebenen Weise, d.h. wird jede Speicherzelle nach dem Umschalten in den Schreibmodus nur ein einziges Mal adressiert, adressiert der Mikroprozessor (5) im Falle der Adressierung des gesamten RAM (51) beginnend mit der Speicherzelle mit der Adressnummer 0 sämtliche Speicherzellen in der Reihenfolge ihrer Adressnummer bis zur Adressnummer 1023. Für den Fall, dass nur einzelne Speichersegmente (51a,51b,51c,51d) adressiert werden gilt dies sinngemäss für die Speicherzellen mit den Adressnummern 0 bis 255, 256 bis 511, 512 bis 667 und 768 bis 1023. Nach dem Umschalten des RAM (51) bzw. eines Speichersegmentes (51a,51b,51c,51d) in den Lesemodus zur telemetrischen Übertragung der gespeicherten Daten wiederholt sich der entsprechende Adressierungsvorgang im Lesemodus.

Im Falle der zweiten beschriebenen Adressierungsweise, die im folgenden für das gesamte RAM (51) beschrieben wird, und für einzelne Speichersegmente (51a,51b,51c,51d) sinngemäss gilt, endet der Adressierungsvorgang im Schreibmodus nicht mit der Adressierung der Speicherzelle mit der Adressnummer 1023. Der Adressierungsvorgang wiederholt sich vielmehr zyklisch bis zu dem Zeitpunkt, zu dem das RAM (51) zur telemetrischen Übertragung der gespeicherten Daten in den Lesemodus geschaltet wird. Unter der Voraussetzung, dass zu diesem Zeitpunkt der Adressierungszyklus bereits einmal vollständig durchlaufen wurde, sind Daten bezüglich der letzten 17 Minuten gespeichert. Wurde als letzte Speicherzelle vor dem Schalten in den Lesemodus beispielsweise die Speicherzelle mit der Adressnummer 563 adressiert, setzt sich der Adressierungszyklus im Lesemodus mit der Speicherzelle mit der Adressnummer 564 zunächst bis zu der Speicherzelle mit der Adressnummer 1023 fort und wird beginnend mit der Speicherzelle mit der Adressnummer 0 bis zur Speicherzelle mit der Adressnummer 563 weitergeführt. Es wurden dann sämtliche Speicherzellen im Lesemodus in der gleichen Reihenfolge wie zuvor im Schreibmodus adressiert, so dass eine vollständige telemetrische Übertragung der gespeicherten Daten erfolgen konnte.

Im Falle des beschriebenen Ausführungsbeispiels ist ein besonderes RAM (51) für die dem zeitlichen Verlauf des Signals des piezoelektrischen Sensors (42) entsprechenden Daten vorgesehen. Dies ist nicht unbedingt erforderlich, da die Speicherung dieser Daten auch in dem Schreib-Lese-Speicher RAM (7) in einem für diesen Zweck reservierten Speicherbereich erfolgen kann.

Der Deutlichkeit halber sind im Falle der FIG 4 die Speichersegmente (51a,51b,51c,51d) als getrennte Speicherbausteine dargestellt. Dies ist nur beispielhaft zu verstehen, da den Speichersegmenten (51a,51b,51c,51d) entsprechende Speichersegmente auch durch Software realisiert werden können.

Im Falle des beschriebenen Ausführungsbeispiels erfolgt ein telemetrischer Datenaustausch zwischen dem Herzschrittmacher (1) und einem PC (36). Es versteht sich, dass es sich bei der externen Datenverarbeitungseinrichtung nicht unbedingt um einen PC handeln muss, sonder dass vielmehr auch andere geeignete Geräte, z.B. sogenannte Programmer, wie sie im Zusammenhang mit Herzschrittmachern gebräuchlich sind, vorgesehen sein können.

Bei dem beschriebenen Ausführungsbeispiel ist als Sensoreinrichtung für die körperliche Aktivität des den Herzschrittmacher (1) tragenden Lebewesens ein piezoelektrischer Sensor (42) mit nachfolgender Signalaufbereitungsschaltung (44) vorgesehen. Ein der körperlichen Aktivität des Lebewesens entsprechendes Signal kann auch auf anderem Wege, z.B. durch die Messung der Körpertemperatur des Lebewesens mittels eines Temperatursensors, erhalten werden.

## Patentansprüche

1. In den Körper eines Lebewesens implantierbares medizinisches Gerät mit Mitteln (3,5,20) zum Stimulieren eines physiologischen Vorgangs des Lebewesens mittels eines Stimulationssignals einstellbarer Stimulationsgröße und selbsttätigen Stellmitteln (5,24) zur Anpassung der Stimulationsgröße an die körperliche Aktivität des Lebewesens, die die Stimulationsgröße anhand eines der körperlichen Aktivität des Lebewesens entsprechenden Signals einer Sensoreinrichtung (42,44,46) einstellen, wobei dem Signal der Sensoreinrichtung (42,44,46) entsprechende Daten von dem medizinischen Gerät zu einer getrennten Datenverarbeitungseinrichtung (36,37,38) telemetrisch übertragbar sind und das Gerät einen wahlweise im Schreib- oder Lesemodus betreibbaren Speicher (51,51a,51b,51c,51d) enthält, dem im Schreibmodus diese Daten zur Speicherung zuführbar sind, welche im Lesemodus zur telemetrischen Übertragung zu der Datenverarbeitungseinrichtung (36,37, 38) abrufbar sind, **dadurch gekennzeichnet**, dass die Daten den zeitlichen Verlauf des Signals der Sensoreinrichtung (42,44,46) während eines definierten Zeitraums darstellen.

2. Gerät nach Anspruch 1, **dadurch gekennzeichnet**, dass der Speicher (51,51a, 51b,51c, 51d) mittels eines dem Gerät telemetrisch zugeführten Signals wahlweise in den Schreib- oder Lesemodus schaltbar ist.

3. Gerät nach Anspruch 1 oder 2, **dadurch gekennzeichnet**, dass der Speicher (51,51a, 51b,51c,51d) eine Vielzahl einzeln adressierbarer Speicherzellen besitzt und dass Adressierungsmittel (5) für die Speicherzellen vorgesehen sind, die nach dem Schalten des Speichers (51,51a,51b,51c,51d) in den Schreibmodus die Speicherzellen in einer definierten Folge jeweils einmal adressieren und nach dem Schalten des Speichers (51,51a,51b,51c,51d) in den Lesemodus die Speicherzellen erneut in der definierten Folge adressieren.

4. Gerät nach Anspruch 1 oder 2, **dadurch gekennzeichnet**, dass der Speicher (51,51a,51b,51c,51d) eine Vielzahl einzeln adressierbarer Speicherzellen besitzt und dass Adressierungsmittel (5) für die Speicherzellen vorgesehen sind, die nach dem Schalten des Speichers (51,51a,51b,51c,51d) in den Schreibmodus die Speicherzellen in einem Adressierungszyklus fortlaufend in einer definierten Folge adressieren und nach dem Schalten des Speichers (51,51a,51b,51c,51d) in den Lesemodus beginnend mit derjenigen Speicherzelle, die der im Schreibmodus zuletzt adressierten Speicherzelle folgt, sämtliche Speicherzellen entsprechend der definierten Folge jeweils einmal adressiert.

5. Gerät nach einem der Ansprüche 1 bis 4, **dadurch gekennzeichnet**, dass der Speicher (50) in mehrere mittels dem Gerät telemetrisch zuführbarer Signale separat ansprechbarer Speichersektoren (51a,51b, 51c,51d) unterteilbar ist, von denen jeder zur Speicherung von dem zeitlichen Verlauf des Signal der Sensoreinrichtung (42,44,46) während jeweils eines definierten Zeitraumes entsprechenden Daten vorgesehen ist.

6. Gerät nach einem der Ansprüche 1 bis 5, **dadurch gekennzeichnet**, dass es als Herzschrittmacher (1) ausgebildet ist, wobei die Mittel (3,5,20) zum Stimulieren eines physiologischen Vorgangs des Lebewesens dessen Herztätigkeit stimulieren und die Stellmittel (5,24) die Stimulationsfrequenz, mit der das Herz (4) im Bedarfsfalle stimuliert wird, an die körperliche Aktivität des Lebewesens anpassen.

7. Gerät nach einem der Ansprüche 1 bis 6, **dadurch gekennzeichnet**, dass die Stellmittel (5,24) mittels der Datenverarbeitungseinrichtung (36,37,38) telemetrisch programmierbar sind.

8. Medizinische Einrichtung umfassend ein Gerät nach einem der Ansprüche 1 bis 7 und eine Datenverarbeitungseinrichtung (36,37,38), **dadurch gekennzeichnet**, dass die Funktion der Stellmittel (5,24) unter Zugrundelegung von dem zeitlichen Verlauf des Signals der Sensoreinrichtung (42,44,46,48) entsprechenden, in dem Speicher (51,51a, 51b,51c,51d) des Gerätes gespeicherten Daten mittels der Datenverarbeitungseinrichtung (36,37,38) zur Ermittlung der Stimulationsgröße in Abhängigkeit von der körperlichen Aktivität des Lebewesens simulierbar ist und dass die Datenverarbeitungseinrichtung (36,37,38) Mittel (38) zur Darstellung der Stimulationsgröße als Funktion der körperlichen Aktivität des Lebewesens aufweist.

## Claims

1. Medical apparatus which can be implanted into the body of a living organism, having means (3, 5, 20) for stimulating a physiological process of the living organism by means of a stimulation signal of settable stimulation magnitude and automatic actuating means (5, 24) for adapting the stimulation magnitude to the physical activity of the living organism, which actuating means set the stimulation magnitude by means of a signal of a sensor device (42, 44, 46), which signal corresponds to the physical activity of the living organism, data corresponding to the signal of the sensor device (42, 44, 46) being capable of being transmitted from the medical apparatus to a separate data processing device (36, 37, 38) by telemetry, and the apparatus including a memory (51, 51a, 51b, 51c, 51d), which can be selectively operated in the write or read mode and to which, in the write mode, these data can be fed for storage, which data, in the read mode, can be called up for transmission, by telemetry, to the data processing device (36, 37, 38), characterized in that the data represent the temporal progression of the signal of the sensor device (42, 44, 46) during a defined period of time.

2. Apparatus according to Claim 1, characterized in that the memory (51, 51a, 51b, 51c, 51d) is selectively switchable into the write mode or read mode by means of a signal fed by telemetry to the apparatus.

3. Apparatus according to Claim 1 or 2, characterized in that the memory (51, 51a, 51b, 51c, 51d) possesses a multiplicity of individually addressable memory cells, and in that addressing means (5) for the memory cells are provided, which, after the switching of the memory (51, 51a, 51b, 51c, 51d) into the write mode, address once in each instance the memory cells in a defined sequence and, after the switching of the memory (51, 51a, 51b, 51c, 51d) into the read mode, address the memory cells afresh in the defined sequence.

4. Apparatus according to Claim 1 or 2, characterized in that the memory (51, 51a, 51b, 51c, 51d) possesses a multiplicity of individually addressable memory cells, and in that addressing means (5) for the memory cells are provided, which, after the switching of the memory (51, 51a, 51b, 51c, 51d) into the write mode, address the memory cells in an addressing cycle progressively in a defined sequence and, after the switching of the memory (51, 51a, 51b, 51c, 51d) into the read mode, address all memory cells in accordance with the defined sequence, once in each instance, commencing with that memory cell which follows the memory cell last addressed in the write mode.

5. Apparatus according to one of Claims 1 to 4, characterized in that the memory (50) can be subdivided into a plurality of memory sectors (51a, 51b, 51c, 51d) which can be separately called by means of signals which can be fed by telemetry to the apparatus, each one of which memory sectors is provided for the storage of data corresponding to the temporal progression of the signal of the sensor device (42, 44, 46) during each respective defined period of time.

6. Apparatus according to one of Claims 1 to 5, characterized in that it is designed as a cardiac pacemaker (1), in which the means (3, 5, 20) for stimulating a physiological process of the living organism stimulate its cardiac activity and the actuating means (5, 24) adapt the stimulation frequency with which the heart (4) is stimulated as required to the physical activity of the living organism.

7. Apparatus according to one of Claims 1 to 6, characterized in that the actuating means (5, 24) are programmable by telemetry by means of the data processing device (36, 37, 38).

8. Medical device comprising an apparatus according to one of Claims 1 to 7, and a data processing device (36, 37, 38), characterized in that the function of the actuating means (5, 24) can be simulated on the basis of data which correspond to the temporal progression of the signal of the sensor device (42, 44, 46, 48) and which are stored in the memory (51, 51a, 51b, 51c, 51d) of the apparatus, by means of the data processing device (36, 37, 38) for the determination of the stimulation magnitude as a function of the physical activity of the living organism, and in that the data processing device (36, 37, 38) exhibits means (38) for the representation of the stimulation magnitude as a function of the physical activity of the living organism.

## Revendications

1. Appareil médical implantable dans le corps d'un être vivant et comportant des moyens (3, 5, 20) destinés à stimuler un processus physiologique de l'être vivant au moyen d'un signal de stimulation ayant une grandeur de stimulation réglable, et des moyens automatiques de réglage (5, 24) destinés à adapter la grandeur de stimulation à l'activité corporelle de l'être vivant et qui règlent la grandeur de stimulation sur la base d'un signal, qui correspond à l'activité corporelle de l'être vivant et qui est envoyé par un dispositif de capteur (42, 44, 46), des données, qui correspondent au signal du dispositif de capteur (42, 44, 46), pouvant être transmises par télémétrie par l'appareil médical à un appareil distinct de traitement de données (36, 37, 38), l'appareil comportant une mémoire (51, 51a, 51b, 51c, 51d), apte à fonctionner au choix dans le mode d'enregistrement ou dans le mode de lecture et à laquelle, dans le mode d'enregistrement, peuvent être envoyées, pour leur mémorisation, ces données qui peuvent être appelées, pendant le mode de lecture, pour la transmission par télémétrie au dispositif de traitement de données (36, 37, 38), caractérisé par le fait que les données représentent la variation dans le temps du signal du dispositif de capteur (42, 44, 46) pendant un intervalle de temps défini.

2. Appareil suivant la revendication 1, caractérisé par le fait que la mémoire (51, 51a, 51b, 51c, 51d) peut être commutée au choix sur le mode d'enregistrement ou sur le mode de lecture au moyen d'un signal envoyé par télémétrie à l'appareil.

3. Appareil suivant la revendication 1 ou 2, caractérisé par le fait que la mémoire (51, 51a, 51b, 51c, 51d) possède une multiplicité de cellules de mémoire adressables individuellement et qu'il est prévu, pour les cellules de mémoire, des moyens d'adressage (5), qui, après passage de la mémoire (51, 51a, 51b, 51c, 51d) au mode d'enregistrement, adressent une fois les cellules de mémoire selon une séquence définie et, après le passage de la mémoire (51, 51a, 51b, 51c, 51d) au mode de lecture, adressent à nouveau les cellules de mémoire selon la séquence définie.

4. Appareil suivant la revendication 1 ou 2, caractérisé par le fait que la mémoire (51, 51a, 51b, 51c, 51d) possède une multiplicité de cellules de mémoire adressables individuellement et qu'il est prévu, pour les cellules de mémoire, des moyens d'adressage (5), qui, après passage de la mémoire (51, 51a, 51b, 51c, 51d) au mode d'enregistrement, adressent les cellules de mémoire au cours d'un cycle d'adressage, d'une manière continue selon une séquence définie, et, après passage de la mémoire (51, 51a, 51b, 51c, 51d) au mode de lecture, adressent respectivement une fois toutes les cellules de mémoire selon la séquence définie, en commençant par la cellule de mémoire, qui succède à la cellule de mémoire adressée en dernier lieu dans le mode d'enregistrement.

5. Appareil suivant l'une des revendications 1 à 4, caractérisé par le fait que la mémoire (50) peut être subdivisée en plusieurs secteurs de mémoire (51a, 51b, 51c, 51d), qui peuvent être appelés séparément au moyen de signaux pouvant être envoyés par télémétrie à l'appareil et dont chacune est prévue pour la mémorisation de données correspondant à la variation dans le temps du signal du dispositif de capteur (42, 44, 46) pendant respectivement un dispositif de capteur (42, 44, 46) pendant respectivement un intervalle de temps défini.

6. Appareil suivant l'une des revendications 1 à 5, caractérisé par le fait qu'il est agencé sous la forme d'un stimulateur cardiaque (1), et que les moyens (3, 5, 20) destinés à stimuler un processus physiologique de l'être vivant, stimulent son activité cardiaque et que les moyens de réglage (5, 24) adaptent la fréquence de stimulation, à laquelle le coeur (4) est stimulé en cas de besoin, à l'activité corporelle de l'être vivant.

7. Appareil suivant l'une des revendications 1 à 6, caractérisé par le fait que les moyens de réglage (5, 24) sont programmables par télémétrie au moyen du dispositif de traitement de données (36, 37, 38).

8. Dispositif médical comprenant un appareil suivant l'une des revendications 1 à 7 et un dispositif de traitement de données (36, 37, 38), caractérisé par le fait que la fonction des moyens de réglage (5, 24) peut être stimulée, en prenant pour base des données qui correspondent à la variation dans le temps du signal du dispositif de capteur (42, 44, 46, 48) et qui sont mémorisées dans la mémoire (51, 51a, 51b, 51c, 51d) de l'appareil, au moyen du dispositif de traitement de données (36, 37, 38) pour la détermination de la grandeur de stimulation en fonction de l'activité corporelle de l'être vivant, et que le dispositif de traitement de données (36, 37, 38) a des moyens (38) destinés à représenter la grandeur de stimulation en fonction de l'activité corporelle de l'être vivant.
